# EUROPEAN PATENT SPECIFICATION

(11) **EP 4 293 049 B1**
(45) Date of publication and mention of the grant of the patent: **20.11.2024**
(21) Application number: 23179582.4
(22) Date of filing: 15.06.2023
(51) Int. Cl.: C08B 11/193

(54) **LIQUID COMPOSITION**
FLÜSSIGE ZUSAMMENSETZUNG
COMPOSITION LIQUIDE

(30) Priority: 16.06.2022 JP 2022097340
(43) Date of publication of application: 20.12.2023
(73) Proprietor: SHIN-ETSU CHEMICAL CO., LTD., Tokyo 1000005 (JP)
(72) Inventor: YOKOSAWA, Takuya, NIIGATA, 942-8601 (JP); MIKI, Kentaro, NIIGATA, 942-8601 (JP)
(74) Representative: Cabinet Nony

(56) References cited:
- EP-A1- 1 803 738
- EP-A1- 2 829 553

## Description

### BACKGROUND OF THE INVENTION

### 1. Field of the Invention

The invention relates to a liquid composition comprising hydroxypropyl methyl cellulose and an organic solvent.

### 2. Related Art

Hydroxypropyl methyl cellulose (hereinafter also referred to as "HPMC") is used for a wide range of applications including coating, controlled release of drugs, and methods of preparing solid dispersions by hot melt extrusion or spray drying with poorly water-soluble drugs.

When HPMC is used in a coating application, it is mostly dissolved in water to form a coating solution. However, when an active ingredient contained in a solid preparation to which the coating solution will be sprayed interacts with water, or when it is necessary to increase a coating rate (i.e., to improve the coating productivity), HPMC is dissolved in a solvent containing at least one organic solvent to form a coating solution. HPMC is also known to be used in a film such as a capsule shell (U.S. Patent No. 3493407, JPH03-279325A, JP2001-506692A, which is a Japanese phase publication of WO 98/27151, and JP2011-500871A, which is a Japanese phase publication of WO 2008/050209A1).

HPMC is further known to be used in a solid dispersion produced by the method comprising steps of: dissolving HPMC in an organic solvent together with a poorly water-soluble drug, and spray-drying the resulting liquid composition.

Generally, prior to coating or spray-drying, the undissolved matter in the composition in which HPMC is dissolved is removed, for example, by filtration with a filer or centrifugation. However, when an amount of undissolved matter is large, there is a problem that clogging of the filter occurs, or the centrifugation requires a prolonged time. Hence, there is a need to improve the solubility of HPMC in an organic solvent.

In order to improve the solubility of HPMC in an organic solvent, it is known to increase a substitution degree (DS) of methoxy groups and a molar substitution (MS) of hydroxypropoxy groups of HPMC. For example, HPMC Methocel^{™} 310 having a high substitution degree (DS) of methoxy groups and a high molar substitution (MS) of hydroxypropoxy groups has been reported (Dow Chemical Company "METHOCHEL Cellulose Ethers Technical Handbook", United States, September 2002, pages 4 to 6)

### SUMMARY OF THE INVENTION

However, when improving the solubility of HPMC in an organic solvent by the method of Dow Chemical Company of Technical Handbook, increase in DS of methoxy groups and MS of hydroxypropoxy groups not only elevates the cost but also changes the physical properties (such as thermal gel properties and adhesiveness) other than the solubility of HPMC. Accordingly, there is a need to improve the solubility of HPMC in an organic solvent without changing DS of methoxy groups and MS of hydroxypropoxy groups.

The invention has been made in view of solving the above problems, and provides HPMC having high solubility in an organic solvent even when DS of methoxy groups and MS of hydroxypropoxy groups of the HPMC are maintained without increase.

As a result of intensive studies, the inventors have found that hydroxypropyl methyl cellulose having a molar substitution (MS) of hydroxypropoxy groups of from 0.1 to 0.5, and a ratio (referred to as "MF at 3-position/MS") of 0.12 or more, as calculated by dividing a molar fraction (referred to as "MF at 3-position") of anhydroglucose units in which only a hydroxy group at the 3-position carbon is substituted with a hydroxypropoxy group and none of hydroxy groups at the 2-position and 6-position carbons is substituted with a hydroxypropoxy group by a molar substitution (referred to as "MS") of hydroxypropoxy groups, has a high solubility in an organic solvent; and completed the invention.

In one aspect of the invention, there is provided hydroxypropyl methyl cellulose having
a molar substitution (MS) of hydroxypropoxy groups of from 0.1 to 0.5;
a ratio (referred to as "MF at 3-position/MS") of 0.12 or more, as calculated by dividing a molar substitution (refers to as "MF at 3-position) of anhydroglucose units in which only a hydroxy group at the 3-position carbon is substituted with a hydroxypropoxy group and none of hydroxy groups at the 2-position and 6-position carbons is substituted with a hydroxypropoxy group by a molar substitution (referred to as "MS") of hydroxypropoxy groups; and
a viscosity at 20°C of from 1.0 to 50 mPa·s, as determined in a 2% by mass aqueous solution.

In another aspect of the invention, there is provided a liquid composition comprising hydroxypropyl methyl cellulose and an organic solvent.

In still another aspect of the invention, there is provided a method for producing a preparation, the method comprising a step of coating a core comprising an active ingredient with the liquid composition.

In a further aspect of the invention, there is provided a method for producing a film, the method comprising steps of: applying the liquid composition on a substrate, and removing the solvent from the liquid composition to obtain a film.

In a still further aspect of the invention, there is provided a method for producing a solid dispersion, the method comprising a step of spray-drying the liquid composition to obtain a solid dispersion.

The molar substitution (MS) of hydroxypropoxy groups means an average mole number of hydroxypropoxy groups per mole of anhydroglucose units.

According to the invention, solubility of HPMC during dissolving the HPMC in an organic solvent is improved, so that clogging during filtering to remove undissolved matter is improved, and the centrifugation time for removing undissolved matter can be shortened.

### DETAILED DESCRIPTION OF THE PREFERRED EMBODIMENTS

### (1) HPMC

First, HPMC will be described.

Regarding HPMC, a ratio (referred to as "MF at 3-position/MS") of a molar fraction (referred to as "MF at 3-position") of the anhydroglucose units in which only a hydroxy group at the 3-position carbon is substituted with a hydroxypropoxy group and none of hydroxy groups at the 2-position and 6-position carbons is substituted with a hydroxypropoxy group to a molar substitution (referred to as "MS") of hydroxypropoxy groups is 0.12 or more, preferably from 0.14 to 0.30, more preferably from 0.16 to 0.25, and still more preferably from 0.16 to 0.20. By using said ratio, a preferable solubility in an organic solvent may be ensured.

Regarding HPMC, a molar fraction (MF at 3-position) of anhydroglucose units in which only a hydroxy group at the 3-position carbon is substituted with a hydroxypropoxy group and none of hydroxy groups at the 2-position and 6-position carbons is substituted with a hydroxypropoxy group means the sum of the average molar fractions (i) to (iv) as defined below.
(i) an average molar fraction of the anhydroglucose units in which among the hydroxy groups at the 2-, 3- and 6-position carbons, each hydroxy group at the 2- and 6-postition carbons is substituted with a methoxy group and a hydroxy group at the 3-positon carbon is substituted with a hydroxypropoxy or methoxypropoxy group.
(ii) an average molar fraction of the anhydroglucose units in which among the hydroxy groups at the 2-, 3- and 6-position carbons, a hydroxy group at the 2-position carbon is substituted with a methoxy group, a hydroxy group at the 6-position carbon is not substituted, and the hydroxy group at the 3-position carbon is substituted with a hydroxypropoxy or methoxypropoxy group.
(iii) an average molar fraction of the anhydroglucose units in which among the hydroxy groups at the 2-, 3- and 6-position carbons, a hydroxy group at the 2-position carbon is not substituted, a hydroxy group at the 6-position carbon is substituted with a methoxy group, and a hydroxy group at the 3-position carbon is substituted with a hydroxypropoxy or methoxypropoxy group.
(iv) an average molar fraction of the anhydroglucose units in which among the hydroxy groups at the 2-, 3- and 6-position carbons, each hydroxy group at the 2- and 6-position carbons is not substituted, and a hydroxy group at the 3-position carbon is substituted with a hydroxypropoxy or methoxypropoxy group.

The molar fraction "MF at 3-position" of HPMC is not particularly limited. It is preferably 0.027 or more, more preferably from 0.027 to 0.050, still more preferably from 0.027 to 0.045, and particularly preferably from 0.040 to 0.045. By using said molar fraction "MF at 3-position", preferable solubility in an organic solvent may be ensured.

Regarding HPMC, the molar fraction (MF at 3-position) of anhydroglucose units in which only a hydroxy group at the 3-position carbon is substituted with a hydroxypropoxy group and none of the hydroxy groups at the 2-position and 6-position carbons is substituted with a hydroxypropoxy group can be determined from the respective detection graph properties identified by using a mass spectrometer and any one of ¹³C-NMR, liquid chromatography and gas chromatography, after HPMC is hydrolyzed in sulfuric acid, then neutralized, filtered, reduced, and further acetylated, as described in Macromolecules, 1987, 20, 2413 and Journal of Fiber Sciences, 1984, 40, T-504.

According to the invention, the molar fraction (MF at 3-position) was determined by the following method.

First, 50 mg of hydroxypropyl methyl cellulose is subjected to addition of 2 ml of a 3% by mass aqueous sulfuric acid solution and hydrolyzed at 140°C for 3 hours to obtain a hydrolysate, and then subjected to addition of 0.8 g of barium carbonate to neutralize the hydrolysate.

Then, the neutralized hydrolysate is dissolved by adding 3mL of methanol, and subjected to centrifugation at 500 G. The supernatant liquid is filtered through a filter having openings of 0.45 µm to collect a filtrate. Then, 3mL of the filtrate is subjected to addition of 120 µL of a solution in which 1.5 g of NaBH₄ is dissolved in 10 mL of 0.2N (i.e., 0.2 mol/L) aqueous sodium hydroxide solution, and the glucose ring is reduced at 37 to 38°C for 1 hour. The resulting mixture is subjected to addition of 100 µL of acetic acid, and then heated at 100°C for 1 hour under atmospheric pressure with nitrogen blowing to evaporate the solvent to obtain a solid. Then, the obtained solid is subjected to addition of 2 mL of pyridine and 1.5 ml of acetic anhydride, is acetylated at 100°C for 1.5 hours, and then subjected to centrifugation at 500 G. The supernatant liquid is filtered through a filter having openings of 0.45 µm to collect a filtrate. A filtrate is heated to 100°C for 1 hour under atmospheric pressure with nitrogen blowing to remove pyridine, acetic anhydride and acetic acid, and then redissolved in 1 ml of diethylene glycol dimethyl ether to obtain a sample. The obtained sample can be subjected to gas chromatography (GC) to measure the molar fraction (MF at 3-position) based on the peak area.

The GC analysis of the sample can be done as follows. To a GC2010 (produced by Shimadzu Corporation) equipped with DB-5 columns (length of 30 m, diameter of 0.25 mm, and film thickness of 0.25 µm), 1µL of the sample solution is injected under the conditions of carrier gas: helium, pressure of helium: 100 kPa, and an inlet temperature: 300°C. The column is held at 150°C for 3 minutes, then heated to 275°C at a rate of 2°C/minute, then heated to 300°C at a rate of 15°C/minute, and then held at 300°C for 5 minutes. The retention time and the peak area of each component is measured by using an FID detector set at 300°C.

The structure corresponding to each detection peak is identified in advance by a mass spectrometer. Each peak of the sample is identified in comparison with the peak identified by the mass spectrometer. A molar fraction (MF at 3-position) of anhydroglurose units in which only a hydroxy group at the 3-position carbon is substituted with a hydroxypropoxy group and none of the hydroxy groups at the 2-position and 6-position carbons is substituted with a hydroxypropoxy group is determined based on a ratio of peak areas. A ratio of the molar fraction (MF at the 3-position) to the molar substitution (MS) of hydroxypropoxy groups is obtained by dividing the former by the latter.

A degree of substitution (DS) of methoxy groups of HPMC is not particularly limited. It is preferably from 1.0 to 2.2, more preferably from 1.5 to 2.2, and still more preferably from 1.7 to 2.2. By using said degree of substitution, preferable solubility in an organic solvent may be ensured. The degree of substitution (DS) of methoxy groups means an average number of methoxy groups per anhydroglucose unit.

The molar substitution (MS) of hydroxypropoxy groups of HPMC is not particularly limited. It is preferably from 0.05 to 0.50, more preferably from 0.10 to 0.40, still more preferably from 0.15 to 0.35, and particularly preferably from 0.18 to 0.35. By using said molar substitution, preferable solubility in an organic solvent and preferable tackiness of the liquid composition may be ensured. The molar substitution (MS) of hydroxypropoxy groups means an average mole number of hydroxypropoxy groups per mole of anhydroglucose unit.

The DS of methoxy groups and the MS of hydroxypropoxy groups of HPMC can be determined by converting the values measured in accordance with the Japanese Pharmacopoeia Seventeenth Edition.

HPMC is preferably 2910 type HPMC having methoxy groups of from 28.0 to 30.0% and hydroxypropoxy groups of from 7.0 to 12.0%, 2906 type HPMC having methoxy groups of from 27.0 to 30.0% and hydroxypropoxy groups of from 4.0 to 7.5%, or 2208 type HPMC having methoxy groups of from 19.0 to 24.0% and hydroxypropoxy groups of from 4.0 to 12.0%, as described in Hypromellose in the Japanese Pharmacopoeia Seventeenth Edition. It is more preferably 2910 type HPMC or 2906 type HPMC, and particularly preferably 2910 type HPMC. By using said above type HPMC, preferably solubility in an organic solvent may be ensured.

On the other hand, when an amount of the hydroxypropoxy groups is excessively high, such as that of type 1828 HPMC having methoxy groups of from 16.5 to 20.0% and hydroxypropoxy groups of from 23.0 to 32.0%, the tackiness of the liquid composition may be high.

A viscosity at 20°C of the 2% by weight aqueous solution of HPMC is from 1.0 to 50 mPa·s, preferably from 1.0 to 15 mPa·s, and more preferably from 2.0 to 8.0 mPa·s. When the viscosity at 20°C of the 2% by mass aqueous solution is less than 1.0 mPa·s, there is a possibility that the coating film becomes brittle. When the viscosity at 20°C of the 2% by mass aqueous solution is more than 50 mPa·s, a viscosity of the coating solution or the solution to be spray-dried becomes high, so that the concentration of the coating solution or the solution to be spray-dried cannot be increased.

When the viscosity at 20°C of the 2.0% by mass aqueous solution of HPMC is 600 mPa·s or more, the viscosity may be determined using a single cylinder-type rotational viscometer in accordance with the viscosity measurement by rotational viscometer of General Tests in the Japanese Pharmacopoeia Seventeenth Edition. When the viscosity at 20°C of the 2.0% by mass aqueous solution of HPMC is less than 600 mPa·s, the viscosity may be determined using a Ubbelohde type viscometer in accordance with the viscosity measurement by capillary tube viscometer of General Tests in the Japanese Pharmacopeia Seventeenth Edition.

Regarding a combination of the ratio (MF at 3-position/MS), the viscosity at 20°C of the 2% by mass aqueous solution and a degree of substitution (DS) of methoxy groups of HPMC, when the ratio (MF at 3-position/MS) is 0.12 or more, the viscosity of HPMC is preferably from 1.0 to 50 mPa·s and the DS is preferably from 1.0 to 2.2.

The ratio (MF at 3-position/MS) is preferably from 0.14 to 0.30. When the ratio (MF at 3-position/MS) is from 0.14 to 0.30, the viscosity of HPMC is more preferably from 1.0 to 15 mPa·s and the DS is more preferably from 1.5 to 2.2. The ratio (MF at 3-position/MS) is more preferably from 0.16 to 0.25. When the ratio (MF at 3-position/MS) is from 0.16 to 0.25, the viscosity of HPMC is still more preferably from 1.0 to 8.0 mPa·s and the DS is still more preferably from 1.7 to 2.2.

### (2) Method for producing HPMC

Next, a method for producing HPMC having a predetermined ratio (MF at 3-position/MS) will be described.

HPMC having a predetermined ratio (MF at 3-position/MS) is produced by the method comprising steps of: for example,
bringing pulp into contact with an alkali metal hydroxide solution to obtain alkali cellulose;
mixing the alkali cellulose with dimethyl ether and a first methylating agent to form a first reaction product mixture;
mixing the first reaction product mixture with a second methylating agent and a hydroxypropylating agent without further incorporation of an alkali metal hydroxide to obtain a second reaction product mixture;
purifying the second reaction product mixture to obtain HPMC;
drying the HPMC to obtain dried HPMC; and
optionally depolmerizing, with an acid, the dried HPMC or the HPMC obtained in the step of purifying the second reaction product mixture;
wherein a reaction percentage of the hydroxypropylating agent is from 50% to 90% at the time when a total reaction percentage of the first and second methylating agents is 50%, regarding a reaction percentage of the hydroxypropylating agent and a total reaction percentage of the first and second methylating agents as 100% at the time when a degree of substitution (DS) of methoxy groups and a molar substitution (MS) of hydroxypropyl groups of hydroxypropyl methyl cellulose in the second reaction product mixture become equal to a degree of substitution (DS) of methoxy groups and a molar substitution (MS) of hydroxy groups of the dried hydroxypropyl methyl cellulose, respectively.

First, a step of bringing pulp into contact with an alkali metal hydroxide solution to obtain alkali cellulose is described.

Examples of the pulp include cellulose pulp such as wood pulp and linter pulp. The pulp may be used in any form such as powder form, sheet form and chip form. The pulp is preferably in sheet form or chip form. By using said form, preferably solubility of HPMC in an organic solvent as well as ease of handling and preferable liquid removal property of alkali cellulose may be ensured.

The intrinsic viscosity of the pulp, which is an indicator of the polymerization degree of the pulp, is preferably from 300 to 2500 ml/g, more preferably from 350 to 2300 ml/g, and still more preferably from 400 to 2000 ml/g. When the intrinsic viscosity is less than 300 ml/g, there is a possibility that the viscosity of the obtained HPMC may become low, and there is a possibility that the washability of the crude HPMC may deteriorate. When the intrinsic viscosity is more than 2500 ml/g, there is a possibility that the obtained HPMC may have inferior solubility in an organic solvent. The intrinsic viscosity of the pulp may be determined in accordance with Method A in JIS P8215.

Examples of the alkali metal hydroxide solution include an aqueous alkali metal hydroxide solution such as an aqueous sodium hydroxide solution or an aqueous potassium hydroxide solution. A concentration of the alkali metal hydroxide in the alkali metal hydroxide solution is preferably from 23 to 60% by mass. By using said concentration, preferable economical efficiency and operability may be ensured.

The mass ratio of the alkali metal hydroxide in the alkali cellulose to the solid component in the pulp (alkali metal hydroxide/solid component in the pulp) is not particularly limited as long as the intended degree of substitution (DS) of methoxy groups and the intended molar substitution (MS) of hydroxypropoxy groups are obtained. It is preferably from 0.30 to 1.50, and more preferably from 0.35 to 1.45.

An amount of the alkali metal hydroxide solution to be used may be appropriately selected in accordance with the above-described mass ratio. The mass of the alkali metal hydroxide component may be calculated based on neutralization titration.

The solid component in the pulp means components other than moisture (i.e., water) in the pulp. The solid component in the pulp includes, in addition to the cellulose as a main component, organic matter such as hemicellulose, lignin and resin, and inorganic matter such as Si and Fe components. The solid component in the pulp may be calculated from the dry matter content determined in accordance with Pulps - Determination of Dry Matter Content in JIS P8203:1998. The dry matter content is the ratio of the mass of the sample being subjected to drying at 105 ± 2°C and reaching constant mass to the mass of the sample prior to the drying. The dry matter content is expressed in % by mass.

Embodiments in which the pulp is brought into contact with the alkali metal hydroxide solution include immersing a pulp sheet in an excess of the alkali metal hydroxide solution. The pulp sheet having sufficiently absorbed the alkali metal hydroxide solution is, for example, pressed to remove the excess alkali metal hydroxide solution and obtain a pulp sheet having a predetermined mass ratio of the alkali metal hydroxide to the solid component in the pulp. The concentration of the alkali metal hydroxide solution to be contacted with the pulp is preferably kept constant in order to stabilize the composition of the alkali cellulose.

In the step of bringing pulp into contact with the alkali metal hydroxide solution, an organic solvent such as dimethyl ether, a methylating agent such as methyl chloride, and a hydroxypropylating agent such as propylene oxide are not present.

Next, the step of mixing the alkali cellulose with dimethyl ether and a first methylating agent to form a first reaction product mixture is described. In a preferable embodiment, the alkali cellulose is subjected to addition of a mixture of dimethyl ether and a first methylating agent, and mixed with stirring to form a first reaction product mixture.

The mass ratio of dimethyl ether to the solid component in the starting pulp (dimethyl ether/solid component in the starting pulp) is preferably from 0.5 to 2.0. By using said mass ratio, preferable solubility of HPMC in an organic solvent may be ensured.

Examples of the first methylating agent include methyl chloride. The mass ratio of the first methylating agent to the solid component in the starting pulp (first methylating agent/solid component in the starting pulp) is preferably from 0.05 to 0.50. By using said mass ratio, preferable solubility of HPMC in an organic solvent may be ensured. The mass ratio of dimethyl ether to the first methylating agent (dimethyl ether/first methylating agent) is preferably from 2.0 to 20.0. By using said mass ratio, preferable solubility of HPMC in an organic solvent may be ensured.

The temperature for the embodiment in which the alkali cellulose is subjected to addition of a mixture of dimethyl ether and a first methylating agent, and mixed with stirring to form a first reaction product mixture, is preferably at 20 to 70°C. By using said temperature, preferably solubility of HPMC in an organic solvent may be ensured. The time for mixing with stirring after addition of the mixture of dimethyl ether and the first methylating agent to the alkali cellulose is preferably from 0.1 to 30 minutes. By using said mixing time, preferably solubility of HPMC in an organic solvent may be ensured.

Next, the step of mixing the first reaction product mixture with a second methylating agent and a hydroxypropylating agent without further incorporation of an alkali metal hydroxide to obtain a second reaction product mixture is described. In a preferable embodiment, the first reaction product mixture is subjected to addition of a second methylating agent and a hydroxypropylating agent without further addition of an alkali metal hydroxide to obtain a second reaction product mixture comprising HPMC as a product.

Examples of the second methylating agent include methyl chloride. The mass ratio of the second methylating agent to the solid component in the starting pulp (second methylating agent/solid component in the pulp) is not limited as long as the intended degree of substitution (DS) of methoxy groups is obtained. It is preferably from 0.9 to 2.5. By using said mass ratio, preferable economical efficiency may be ensured. The first methylating agent and the second methylating agent may be of the same type or of different types. They are preferably of the same type. By using the same type, preferable ease of purifying HPMC may be ensured.

The mass ratio of the first methylating agent to the second methylating agent (first methylating agent to second methylating agent) if preferably from 0.01 to 0.20. By using said mass ratio, preferably solubility of HPMC in an organic solvent may be ensured.

Examples of the hydroxypropylating agent include propylene oxide. The mass ratio of the hydroxypropylating agent to the solid component in the starting pulp (hydroxypropylating agent/solid component in the starting pulp) is not limited as long as the intended molar substitution (MS) of hydroxypropoxy groups is obtained. It is preferable to use the hydroxypropylating agent so as to make the mass ratio to be from 0.2 to 1.5. By using said mass ratio, preferable economical efficiency may be ensured.

The second methylating agent and the hydroxypropylating agent may be added simultaneously to the first reaction product mixture, or either the second methylating agent or the hydroxypropylating agent may be preferentially added to the first reaction product mixture.

The anhydroglucose unit of cellulose has three substitutable hydroxy groups. Regarding the methylation, in general, the hydroxy group at the 2-position carbon of the glucose anhydride unit is the most reactive, the hydroxy group at the 6-position carbon is less reactive, and the hydroxy group at the 3-position carbon is the least reactive. Regarding the hydroxypropylation, in general, the hydroxy group at the 6-position carbon of the anhydroglucose unit is the most reactive, and the reactivity of each hydroxy group at the 2-and 3-positions carbons is less reactive. Regarding the hydroxypropylation, the reactivity of the hydroxy group at the 2-position carbon is the same as the reactivity of the hydroxy group at the 3-position carbon. Hence, in order to increase the ratio of the molar fraction (MF at 3-position) to MS, hydroxypropylation of each hydroxy group at the 2- and 6-position carbons is suppressed, and hydroxypropylation of the hydroxy group at the 3-position carbon is allowed to proceed.

One of the methods for increasing the ratio (MF at 3-position/MS) includes controlling a reaction percentage of the hydroxypropylating agent relative to a total reaction percentage of the first and second methylating agents. A reaction percentage of the hydroxypropylating agent is from 50% to 90% at the time when a total reaction percentage of the first and second methylating agents is 50%, regarding a reaction percentage of the hydroxypropylating agent and a total reaction percentage of the methylating agents as 100% at the time when a final degree of substitution (DS) of methoxy groups and a final molar substitution (MS) of hydroxypropoxy groups of HPMC are obtained. By using said reaction percentages, preferable control of the ratio (MF at 3-position/MS) may be ensured.

Here, "total reaction percentage of the methylating agents" refers to the total reaction percentage (expressed as a percentage) of the methylating agents at an arbitrary time point, regarding the total reaction percentage as 100% when the methylating agents are reacted in such a molar amount (equimolar amount) that the molar amount of the alkali metal hydroxide in the alkali cellulose becomes equal to the molar amount of methoxy groups contributing to the methylation. For example, the total reaction percentage of the methylating agents in the case of using methyl chloride as the first and second methylating agents is a ratio (expressed as percentage) of the reacted molar amount of methyl chloride at an arbitrary time point to the same molar amount (equimolar amount) of the methylating agents as the molar amount of the alkali metal hydroxide in the alkali cellulose. It is because the alkali metal hydroxide in the alkali cellulose is consumed in a molar amount equal to the reacted molar amount of methyl chloride.

When methylation is carried out using a methylating agent and an alkali metal hydroxide, the methylating agent is used typically in a molar amount equal to or more than the molar amount of the alkali metal hydroxide in order to enhance the efficiency of methylation. However, the excess molar amount of the methylating agent, which exceeds the molar amount of the alkali metal hydroxide, is irrelevant to the calculation of the reaction percentage of the methylating agent.

The term "reaction percentage of the hydroxypropylating agent" means the ratio of the molar amount of the hydroxypropyl groups generated through the hydroxypropylation by the hydroxypropylating agent at an arbitrary time point to the overall molar amount of the hydroxypropyl groups generated through the hydroxypropylation, and is expressed as a percentage.

Although the methylation is based on a molar amount equal to a molar amount of alkali metal hydroxide in alkali cellulose, the hydroxypropylation is based on the overall molar amount of hydroxypropyl groups generated through the hydroxypropylation by the hydroxypropylating agent. It is because, for example, after propylene oxide is reacted as the hydroxypropylating agent, the propylene oxide is changed into a hydroxypropyl group and acts as an alkoxide, so that the alkali metal hydroxide derived from alkali cellulose functions as a catalyst, and the reaction between the hydroxypropylating agent and the alkali metal hydroxide does not proceed stoichiometrically.

The target of the methylation and hydroxypropylation is not limited to O-alkali metal (e.g., -ONa) in alkali cellulose, but includes water in a reaction system, methanol generated by the reaction of a methylating agent with an alkali metal hydroxide derived from alkali cellulose and/or water, and propylene glycol generated by the reaction of a hydroxypropylating agent with an alkali metal hydroxide or water.

The total reaction percentage of the methylating agents and the reaction percentage of the hydroxypropylating agent may be determined by a method comprising steps of quickly recovering the methylating agent or the hydroxypropylating agent from the reactor to determine the amount of the methylating agent or hydroxypropylating agent remaining in the reactor at an arbitrary time point, and diving the reacted amount of the methylating agent or hydroxypropylating agent at the arbitrary time point by the overall amount of the methylating agent or the hydroxypropylating agent to be added into the reactor. However, the total reaction percentage of the methylating agents is calculated using the molar amount obtained by subtracting the excess molar amount from the recovered molar amount after the recovered molar amount becomes equal to or less than the molar amount obtained by subtracting the excess molar amount from the blended molar amount, regarding the total reaction percentage of the methylating agents as 100% at the time when the methylating agent is reacted in such a molar amount (equimolar amount) that the molar amount of the alkali metal hydroxide in the alkali cellulose becomes equal to the molar amount of methyl groups contributing to the methylation.

The total reaction percentage of the methylating agents and the reaction percentage of the hydroxypropylating agent may also be obtained by using a simulation based on a chemical reaction rate equation obtained by an experiment.

When the first reaction product mixture.is blended with the second methylating agent and the hydroxypropylating agent, it is preferable to add the second methylating agent and the hydroxypropylating agent to the first reaction product mixture.

The blending time of the second methylating agent is not particularly limited as long as the reaction percentage of the hydroxypropylating agent to the total reaction percentage of the first and second methylating agents can be controlled. It is preferably from 5 to 100 minutes, and more preferably from 5 to 90 minutes. The blending time of the hydroxypropylating agent is not particularly limited as long as the reaction percentage of the hydroxypropylating agent to the total reaction percentage of the first and second methylating agents can be controlled, it is preferably from 5 to 80 minutes, and more preferably from 10 to 60 minutes.

The temperature of the etherification reaction after adding the second methylating agent and the hydroxypropylating agent is not particularly limited as long as the reaction percentage of the hydroxypropylating agent to the total reaction percentage of the first and second methylating agents can be controlled. It is preferably from 55 to 110°C. The temperature of the etherification reaction may be variable or constant within the above range.

Next, a step of purifying the second reaction product mixture to obtain HPMC is described. This step is for isolating HPMC from the second reaction product mixture. For example, the second reaction product mixture is washed, and then subjected to liquid removal such as filtering or pressing to separate HPMC from the second reaction product mixture.

The washing can be carried out by using, for example, water, or a mixture of water and an organic solvent (e.g., acetone). The temperature of the water or the mixture of water and an organic solvent to be used for the washing is preferably from 60 to 100°C.

The water content in the washed HPMC is preferably from 25 to 95% by mass, and more preferably from 35 to 95% by mass. By using said water content, preferable impurity removal and preferable adjustment of the water in the subsequent step may be ensured.

Next, a step of drying the HPMC obtained by the purification to obtain dried HPMC is described.

The drying can be conducted, for example, by using a dryer. Examples of the dryer include a fan dryer. Examples of the drying type include a hot air type, a conductive heating type, and a combination of these types. The drying temperature is preferably from 60 to 120°C. By using said drying temperature, preferable drying efficiency may be ensured. The drying time is preferably from 3 to 24 hours. By using said drying time, the preferable productivity may be ensured.

The dried HPMC may be subjected to optional pulverization. The pulverization is not particularly limited. Example of the pulverizer include an impact pulverization apparatus for pulverizing a material through collision of the material each other or collision of the material with a substrate; and a ball mill or roller mill for pulverizing a material by interposing the material between substrates.

An optional step of depolymerizing, with an acid, the dried HPMC or the HPMC (before drying) obtained in the step of purifying the second reaction product mixture, is described. When the pulverization also is carried out, the depolymerization with an acid may be carried out before or after the pulverization.

Examples of the acid include a hydrogen halide such as hydrogen chloride. The acid may be used as an aqueous acid solution or the like. For example, hydrogen chloride may be used as hydrochloric acid (aqueous hydrogen chloride). The concentration of hydrogen chloride in the aqueous hydrogen chloride solution is preferably from 1 to 45% by mass. An amount of the acid to be used is preferably from 0.04 to 1.00 parts by mass relative to 100 parts by mass of HPMC.

The reaction temperature of the depolymerization with an acid is preferably from 40 to 85°C. The reaction time of the depolymerization with an acid is preferably from 0.1 to 4 hours. After the depolymerization with an acid is completed, the acid may be removed by, for example, reducing the inside pressure of the reactor. Optional sodium bicarbonate or the like may be added to neutralize the acid used for the depolymerization.

### (3) Liquid Composition

The liquid composition comprises the above-described HPMC, and an organic solvent. The liquid composition contains HPMC dissolved in the organic solvent. However, an optional component or components, other than the HPMC, may be dissolved or dispersed in the organic solvent. The liquid composition is preferably in the form of a solution.

Examples of the organic solvent include, but are not limited to, methanol, ethanol, isopropyl alcohol, benzyl alcohol, acetone, ethyl acetate, toluene, xylene, ethylbenzene, propylbenzene, butylbenzene, 1,2,4-trimethylbenzene, diisopropylbenzene, diethylbenzene, methylisopropylbenzene, chloroform, dichloromethane, N-methyl-2-pyrrolidone, N-ethyl-2-pyrrolidone, dimethyl sulfoxide and dimethylformamide. Among these organic solvents, methanol, ethanol, isopropyl alcohol, acetone and dichloromethane are preferable. The organic solvent may be used singly or a mixed solvent of two or more. It is preferable to use the mixed solvent of two or more organic solvents, or the later-described mixed solvent of an organic solvent and water.

The content of HPMC in the liquid composition is not particularly limited. It is preferably more than 0% by mass and not more than 20% by mass, more preferably from 3.0 to 16.0% by mass, and still more preferably from 4.5 to 16.0% by mass. When the content of HPMC is more than 20.0% by mass, a viscosity of the coating solution or the solution to be spray-dried becomes high, so that there is a possibility that the spray mist is coarsened or the spray itself cannot be carried out.

In addition to HPMC and an organic solvent, the liquid composition may comprise optional water. The type of water is not particularly limited. Examples of the water include purified water, ion-exchanged water, distilled water, and tap water.

When the liquid composition contains water, the ratio of the organic solvent to the water is not particularly limited. The ratio of the organic solvent to a total of the solvents is preferably from 50.0 to 99.5% by mass, more preferably from 60.0 to 90.0% by mass, and particularly preferably from 70.0 to 80.0% by mass. When the ratio of the organic solvent to a total of the solvents is less than 50.0% by mass, the drying speed of the liquid composition used for coating or spray-drying becomes slow. Consequently, there is a possibility that the spray rate of the coating or spray-drying cannot be increased.

The liquid composition may comprise an optional active ingredient in addition to HPMC and an organic solvent.

The active ingredient is not particularly limited as long as it is an orally administrable. Examples of the active ingredient include drugs used in pharmaceuticals, and active ingredients used in health foods such as foods with nutrient function claims, foods for specified health uses, and foods with functional claims.

Example of the drug used in pharmaceutical products include a drug for the central nervous system, a drug for the cardiovascular system, a drug for the respiratory system, a drug for the digestive system, an antibiotic, an antitussive and expectorant, an antihistamine, an antipyretic anti-inflammatory analgesic, a diuretic, an autonomic agent, an antimalarial agent, an antidiarrheal agent, a psychotropic,and vitamins and derivatives thereof.

Examples of the drug for the central nervous system include diazepam, idebenone, naproxen, piroxicam, indomethacin, sulindac, lorazepam, nitrazepam, phenytoin, acetaminophen, ethenzamide, ketoprofen, and chlordiazepoxide.

Examples of the drug for the cardiovascular system include molsidomine, vinpocetine, propranolol, methyldopa, dipyridamole, furosemide, triamterene, nifedipine, atenolol, spironolactone, metoprolol, pindolol, captopril, isosorbide dinitrate, delapril hydrochloride, meclofenoxate hydrochloride, diltiazem hydrochloride, etilefrine hydrochloride, digitoxin, and alprenolol hydrochloride.

Examples of the drug for the respiratory system include amlexanox, dextromethorphan, theophylline, pseudoephedrine, salbutamol, and guaifenesin.

Examples of the drug for the digestive system include a benzimidazole drug having antiulcer action, such as 2-[[3-methyl-4-(2,2,2-trifluoroethoxy)-2-pyridyl]methylsulfinyl]benzimidazole and 5-methoxy-2-[(4-methoxy-3,5-dimethyl-2-pyridyl)methylsulfinyl]benzimidazole; cimetidine; ranitidine; pirenzepine hydrochloride; pancreatin; bisacodyl; and 5-aminosalicylic acid.

Examples of the antibiotic include talampicillin hydrochloride, bacampicillin hydrochloride, cefaclor, and erythromycin.

Examples of the antitussive and expectorant include noscapine hydrochloride, carbetapentane citrate, isoaminile citrate, and dimemorfan phosphate.

Examples of the antihistamine include chlorpheniramine maleate, diphenhydramine hydrochloride, and promethazine hydrochloride.

Examples of the antipyretic anti-inflammatory analgesic include ibuprofen, diclofenac sodium, flufenamic acid, sulpyrine, aspirin, and ketoprofen.

Examples of the diuretic include caffeine.

Examples of the autonomic agent include dihydrocodeine phosphate, dl-methylephedrine hydrochloride, atropine sulfate, acetylcholine chloride, and neostigmine.

Examples of the antimalarial agent include quinine hydrochloride.

Examples of the antidiarrheal agent include loperamide hydrochloride.

Examples of the psychotropic include chlorpromazine.

Examples of the vitamins and derivatives thereof include vitamin A, vitamin B1, fursultiamine, vitamin B2, vitamin B6, vitamin B12, vitamin C, vitamin D, vitamin E, vitamin K, calcium pantothenate, and tranexamic acid.

Examples of the active ingredient used in the health food include the above vitamins and derivatives thereof, minerals, carotenoids, amino acids and derivatives thereof, plant extracts, and health food materials.

Examples of the mineral include calcium, magnesium, manganese, zinc, iron, copper, selenium, chromium, sulfur, and iodine.

Examples of the carotenoid include β-carotene, α-carotene, lutein, cryptoxanthin, zeaxanthin, lycopene, astaxanthin, and multicarotene.

Examples of the amino acid include an acidic amino acid, a basic amino acid, a neutral amino acid, and an acidic amino acid amide.

Examples of the acidic amino acid include aspartic acid and glutamic acid.

Examples of the basic amino acid include lysine, arginine, and histidine.

Examples of the neutral amino acid include linear aliphatic amino acids such as alanine and glycine; branched aliphatic amino acids such as valine, leucine and isoleucine; hydroxyamino acids such as serine and threonine; sulfur-containing amino acids such as cysteine and methionine; aromatic amino acids such as phenylalanine and tyrosine; heterocyclic amino acids such as tryptophan; and imino acids such as proline.

Examples of the acidic amino acid amide include asparagine and glutamine.

Examples of the amino acid derivative include acetylglutamine, acetylcysteine, carboxymethylcysteine, acetyltyrosine, acetylhydroxyproline, 5-hydroxyproline, glutathione, creatine, S-adenosylmethionine, glycylglycine, glycylglutamine, dopa, alanylglutamine, carnitine and γ-aminobutyric acid.

Examples of the plant extract include aloe extract, propolis extract, agaricus extract, Panax ginseng extract, ginkgo leaf extract, turmeric extract, curcumin, sprouted brown rice extract, shiitake mycelium extract, Rubus suavissimus extract, sweet Hydrangea leaf extract, Fomes yucatensis extract, sesame extract, garlic extract, maca (Lepidium meyenii) extract, plant worm (Cordyceps sinensis) extract, camomile extract, and red pepper extract.

Examples of the health food material include royal jelly; dietary fibers; proteins; bifidobacteria; lactic acid bacteria; chitosan; yeast; glucosamine; lecithin; polyphenols; cartilage of animals, fish and shellfish; soft-shelled turtle; lactoferrin; freshwater clams; eicosapentaenoic acid; germanium; enzymes; creatine; carnitine; citric acid; raspberry ketone; coenzyme Q10; methylsulfonylmethane; and soybean peptides bonded with phospholipids.

Particularly using the HPMC in accordance with the invention as a carrier of a solid dispersion containing a poorly water-soluble drug, solubility of the poorly water-soluble drug may be improved. Poorly water-soluble drugs refer to drugs listed in the Japanese Pharmacopoeia Seventeenth Edition as "slightly soluble", "very slightly soluble", or "practically insoluble or insoluble" in water. The term "slightly soluble" means that 1 g or 1 mL of a solid pharmaceutical product dissolves in 100 mL or more and less than 1000 mL of water within 30 minutes when placed in a beaker and shaken vigorously at 20 ± 5°C for 30 seconds every 5 minutes. The term "very slightly soluble" means that 1 g or 1 mL of a solid pharmaceutical product dissolves in 1000 mL or more and less than 10,000 mL of water within 30 minutes in the same manner. The term "practically insoluble or insoluble" means that 1 g or 1 mL of a solid pharmaceutical product dissolves in 10000 mL or more of water within 30 minutes.

In the above test of pharmaceutical product, dissolution of a poorly water-soluble drug means that the drug dissolves or becomes miscible in water, and also means that fibers, etc. are found to be un-present or present at a very slight amount.

Examples of the poorly water-soluble drugs include azole-based compounds such as itraconazole, ketoconazole, fluconazole and miconazole; dihydropyridine-based compounds such as nifedipine, nitrendipine, amlodipine, nicardipine, nilvadipine, ferrodipine and efonidipine; propionic acid-based compounds such as ibuprofen, ketoprofen and naproxen; indoleacetic acid-based compounds such as indomethacin and acemetacin; griseofulvin; phenytoin; carbamazepine; and dipyridamole.

The viscosity of the liquid composition is not particularly limited. It is preferably from 1 to 10000 mPa·s, more preferably from 1 to 1000mPa·s, and particularly preferably from 1 to 200 mPa·s. By using said viscosity, preferable spraying of the liquid composition during coating or spray-drying may be ensured. The viscosity of the liquid composition may be measured by using a single cylinder-type rotational viscometer in accordance with the viscosity measurement by rotational viscometer of General Tests in the Japanese Pharmacopoeia Seventeenth Edition.

### (4) Preparation and Film Obtained by Coating

The preparation can be produced by a method comprising a step of coating a core comprising an active ingredient with the liquid composition.

A method for producing a preparation by coating is not particularly limited. It is possible to coat a core particle containing a drug, or a solid preparation such as a drug or active ingredient with the liquid composition which is a coating solution. Examples of the solid preparation include tablets, granules, fine granules, and capsules. The examples also include orally rapidly disintegrating tablets.

The solid preparation may contain an optional additive which is commonly used in this field. Examples of the optional additive include an excipient, a binder, a disintegrant, a lubricant, an aggregation prevention agent, and a solubilizing agent for a pharmaceutical compound.

Examples of the excipient include sugars such as sucrose, lactose and glucose; sugar alcohols such as mannitol, sorbitol and erythritol; starch; crystalline cellulose; calcium phosphate; and calcium sulfate.

Examples of the binder include polyvinyl alcohol, polyacrylic acid, polymethacrylic acid, polyvinylpyrrolidone, glucose, white sugar, lactose, maltose, dextrin, sorbitol, mannitol, hydroxyethyl cellulose, hydroxypropyl methyl cellulose, hydroxypropyl cellulose, macrogols, gum arabic, gelatin, agar, and starch.

Examples of the disintegrant include low-substituted hydroxypropyl cellulose, carmellose or a salt thereof, croscarmellose sodium, sodium carboxymethyl starch, crospopidone (polyvinylpyrrolidone), crystalline cellulose, and crystalline cellulose with carmellose sodium.

Examples of the lubricant and the aggregation prevention agent include talc, magnesium stearate, calcium stearate, colloidal silica, stearic acid, waxes, hydrogenated oils, polyethylene glycols, and sodium benzoate.

Examples of the solubilizing agent for a pharmaceutical compound include organic acids such as fumaric acid, succinic acid, malic acid and adipic acid.

The solid preparation may be produced, for example, by a method comprising a step of tableting a drug and the above-mentioned additive to obtain a tablet, or a step of granulating a drug and the above-mentioned additive to obtain granules or fine granules.

The liquid composition as a coating solution may comprise, in addition to HPMC and an organic solvent, an optional additive such as a plasticizes, a pigment, a colorant, talc, an adhesion preventing agent, an antifoaming agent, and a fragrance.

Examples of the plasticizer include polyethylene glycol, glycerin, propylene glycol, triacetin, and triethyl citrate, and preferable examples include polyethylene glycol and glycerin. By using the preferable plasticizer, excellent compatibility with HPMC may be ensured. When a composition contains a large amount of pigment, the content of the plasticizer is typically increased. In general, an amount of the plasticizer is preferably from 3 to 50% by mass relative to the amount of HPMC.

Examples of the pigment include titanium oxide, aluminum lake, and an edible dye. An amount of the pigment varies depending on the intended light shielding or coloring. It is preferably within a range of from 0.1 to 30% by mass relative to the amount of HPMC.

Examples of the adhesion preventing agent include solid polyethylene glycol, aerosil (silicon dioxide), and titanium dioxide.

Examples of the antifoaming agent include a silicone-based antifoaming agent such as KM-72 (produced by Shin-Etsu Chemical Co., Ltd.), a polyoxyethylene polyoxypropylene glycol-based antifoaming agent such as Pluronic^{®} F68 (produced by ADEKA), and sorbitan sesquioleate.

Examples of the fragrance include spearmint and menthol.

Each amount of the colorant, the talc, the adhesion preventing agent, the antifoaming agent, and the fragrance may be the same as the amount generally used in a coating composition.

Further, the liquid composition may comprise an optional other coating base as long as the effects of the present invention are not hindered. Examples of the optional other coating base include water-soluble vinyl derivatives such as polyvinylpyrrolidone and polyvinyl alcohol; acrylic acid-based copolymers such as methacrylic acid copolymer LD, an ethyl acrylate-methyl methacrylate copolymer dispersion liquid; and cellulose derivatives such as ethyl cellulose, hydroxypropyl cellulose, hydroxypropyl methyl cellulose, hydroxypropyl methyl cellulose phthalate and hydroxypropyl methyl cellulose acetate succinate.

The coating solution may be produced by a method of adding HPMC to a single organic solvent, a method of adding HPMC to two or more organic solvents, or a method of adding HPMC to a mixed solvent of water and an organic solvent such as ethanol or methanol. When HPMC is added to a mixed solvent, it is preferable to add the HPMC to an organic solvent to disperse the HPMC therein, and then add water to dissolve the HPMC. By using said addition order, the preferable aggregation prevention may be ensured.

Further, the above-described optional additive such as a plasticizer may be added to the coating solution to produce a coating solution.

A coating apparatus is not particularly limited. Examples of the coating apparatus may include a pan coating apparatus, a fluidized bed coating apparatus, a tumbling fluidized bed coating apparatus, a Wurster fluidized bed, and a hybrid fluidized bed.

### (5) Film Obtained by Coating

A film such as a capsule shell may be produced by a method comprising steps of: applying the liquid composition on a substrate and removing the solvent from the applied liquid composition to obtain a film. Further, the method may comprise an optional step of removing the film from the substrate.

A method for producing a film such as a capsule shell is not particularly limited. Examples of the method include those described in the above-mentioned US 3493407, JPH03-279325A, JP 2001-506692A and JP 2011-500871A.

### (6) Solid dispersion

A solid dispersion may be produced by a method comprising a step of spray-drying a liquid composition comprising HPMC, an organic solvent, and further an active ingredient to obtain a solid dispersion.

The method for producing the solid dispersion is not particularly limited. The solid dispersion may be produced by a method comprising a step of solvent removal or precipitation from a solution to be subjected to the spray-drying, if needed, wherein the solution is obtained by adding an active ingredient to a liquid composition. The solution to be subjected to the spray-drying is preferably a homogeneous solution in which an active ingredient (e.g., a drug) and HPMC are more uniformly dissolved. Examples of the solvent removal include evaporation to dryness and spray-drying.

The spray-drying broadly refers to a method in which a solution containing an active ingredient is divided into small droplets (spray) and the solvent is rapidly removed from the droplets by evaporation. The driving force for removing the solvent is generally obtained by making the partial pressure of the solution lower than the vapor pressure of the solvent at the temperature at which the droplets are dried. In a preferable embodiment, the droplets are mixed with a hot drying gas, or partial vacuum is maintained in a solvent-removal device.

A solution containing an active ingredient, HPMC and a solvent may be spray-dried through a variety of nozzle mechanisms. For example, various types of nozzles may be used. Preferable examples of the nozzle include a two-fluid nozzle, a fountain nozzle, a flat fan nozzle, a pressure nozzle, and a rotary atomizer.

The solution to be subjected to the spray-drying may be delivered at a wide range of flow rates and temperatures. When pressurized during spraying, it is possible to spray at a wide range of pressures. In general, as the specific surface area of the droplets increases, the rate of solvent evaporation increases. For this reason, the droplet at the departure from the nozzle is preferably less than 500 µm, more preferably less than 400 µm, and still more preferably from 5 to 200 µm. A combination of flow rate, temperature and pressure, capable of forming said droplet, is preferable. The solution after the departure from the nozzle rapidly solidifies.

The sprayed solution rapidly solidifies to become a solid dispersion. The solidified solid dispersion generally remains in the spray-drying chamber for about 5 to 60 seconds during which the solvent is being removed from the solid powder. Regarding the temperature during the spray-drying, an inlet temperature is preferably from about 20°C to 150°C, while an outlet temperature is preferably from about 0°C to 85°C.

A smaller residual solvent content in the solid dispersion is preferable. It is because mobility of the active ingredient molecules in the amorphous solid dispersion is suppressed, thereby increasing the stability. When further removal of the residual solvent is required, secondary drying may be carried out. Suitable examples of the secondary drying include tray drying, fluidized bed drying, belt drying, and microwave drying.

The liquid composition as a solution to be subjected to the spray-drying may comprise, in addition to HPMC and an organic solvent, an optional additive such as a plasticizer, a pigment, a colorant, talc, an anti-adhesion agent, an antifoaming agent and a flagrance, including the same examples as described in the coating solution.

### EXAMPLES

Hereinafter, the invention is described in detail with reference to Synthesis Examples, Comparative Synthesis Examples, Examples, and Comparative Examples.

### Synthesis Example 1

Pulp chips being derived from wood and having an intrinsic viscosity of 520 ml/g was immersed in a 49% by mass aqueous sodium hydroxide solution, and then an excess of the 49% by mass aqueous sodium hydroxide solution was removed to obtain alkali cellulose. The mass ratio of the sodium hydroxide in the obtained alkali cellulose to the solid component in the pulp (sodium hydroxide/solid component in the pulp) was 1.25.

The 19.9 kg (5.5 kg of solid component in the pulp) of the obtained alkali cellulose was placed in a pressure vessel (i.e., reactor, an internal volume of 100L) with a plowshare-type internal stirring blade. The pressure in the pressure vessel was reduced to -97 kPa, and then nitrogen gas was introduced to let the pressure return to atmospheric pressure. Then, the pressure was reduced to -97 kPa again.

Next, a mixture of 2.75 kg of dimethyl ether and 0.48 kg of first methyl chloride was added into the reactor using a pressure pump, and stirred at 60°C for 10 minutes to obtain a first reaction product mixture. Subsequently, 10.9 kg of second methyl chloride was added into the reactor for 50 minutes. Simultaneously with the start of the addition of the second methyl chloride, the addition of propylene oxide into the reactor was started using a pressure pump. The 2.86 kg of the propylene oxide was added into the reactor for 12 minutes. The inside temperature of the reactor at the beginning of adding the second methyl chloride was 60°C, and increased to 100°C for completing the reaction for a total of 2 hours to obtain crude HPMC (second reaction product mixture). In a separate experiment for sampling under the same conditions, the reaction percentage of the propylene oxide was 83.5% at the time when the total reaction percentage of the first and second methyl chlorides was 50%.

The obtained crude HPMC was washed with hot water of 95°C. Then, the washed HPMC was subjected to liquid removal by a centrifugal dehydrator, dried at 70°C for 18 hours using a fan dryer, and pulverized with a small Wiley mill. The viscosity at 20°C of the 2% by mass aqueous solution of the obtained HPMC was 1000 mPa·s, as determined using a single cylinder-type rotational viscometer in accordance with the viscosity measurement by rotational viscometer of General Tests in the Japanese Pharmacopoeia Seventeenth Edition.

The 1 kg of the obtained HPMC was placed in a 20L Henschel mixer, and stirred at 200 rpm, while spraying 12% by mass hydrochloric acid thereto. The hydrochloric acid was sprayed in such an amount that 0.3 parts by mass of HCl was added relative to 100 parts by mass of HPMC. The 500 g of the resulting mixture was transferred into a 2 L glass reactor and was rotated in a water bath of 80°C for 60 minutes for depolymerization. Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

### Synthesis Example 2

HPMC was synthesized, pulverized and depolymerized in the same manner as in Synthesis Example 1 except that the added amount of the propylene oxide was 4.73 kg, the addition time of the second methyl chloride was 30 minutes, and the addition time of the propylene oxide was 50 minutes.

In a separate experiment for sampling under the same conditions, the reaction percentage of the propylene oxide was 73.4% at the time when the total reaction percentage of the first and second methyl chlorides was 50%. Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

### Synthesis Example 3

HPMC was synthesized, pulverized and depolymerized in the same manner as in Synthesis Example 2 except that the time of depolymerization was changed to 90 minutes.

Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

### Synthesis Example 4

HPMC was synthesized, pulverized and depolymerized in the same manner as in Synthesis Example 2 except that the time of depolymerization was changed to 200 minutes.

Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

### Synthesis Example 5

Pulp chips being derived from wood and having an intrinsic viscosity of 520 ml/g was immersed in a 49% by mass aqueous sodium hydroxide solution, and then an excess of the 49% by mass aqueous sodium hydroxide solution was removed to obtain alkali cellulose. The mass ratio of the sodium hydroxide in the obtained alkali cellulose to the solid component in the pulp (sodium hydroxide/solid component in the pulp) was 1.17.

The 19.0 kg (5.5 kg of solid component in the pulp) of the obtained alkali cellulose was placed in a pressure vessel (i.e., reactor, an internal volume of 100 L) with a plowshare-type internal stirring blade. The pressure in the pressure vessel was reduced to -97 kPa, and then nitrogen gas was introduced to let the pressure return to atmospheric pressure. Then, the pressure was reduced to -97 kPa again.

Next, a mixture of 2.75 kg of dimethyl ether and 0.48 kg of first methyl chloride was added to the reactor using a pressure pump, and stirred at 60°C for 10 minutes to obtain a first reaction product mixture. Subsequently, 10.5 kg of second methyl chloride was added into the reactor for 30 minutes. At the same when the addition of the second methyl chloride to the reactor was started, the addition of propylene oxide into the reactor was started using a pressurization pump. The 3.01 kg of propylene oxide was added into the reactor for 50 minutes. The inside temperature of the reactor at the beginning of adding the second methyl chloride was 60°C, and increased to 100°C for completing the reaction for a total of 2 hours to obtain crude HPMC (second reaction product mixture). In a separate experiment for sampling under the same conditions, the reaction percentage of the propylene oxide was 60.5% at the time when the total reaction percentage of the first and second methyl chlorides was 50%.

The obtained crude HPMC was washed with hot water of 95°C. Then, the washed HPMC was subjected to liquid removal by a centrifugal dehydrator, dried at 70°C for 18 hours using a fan dryer, and pulverized with a small Wiley mill. The viscosity at 20°C of the 2% by mass aqueous solution of the obtained HPMC was 1000 mPa·s, as determined using a single cylinder-type rotational viscometer in accordance with the viscosity measurement by rotational viscometer of General Tests in the Japanese Pharmacopoeia Seventeenth Edition.

The obtained HPMC was depolymerized under the same conditions as in Synthesis Example 3. Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

### Comparative Synthesis Example 1

HPMC was synthesized, pulverized and depolymerized in the same manner as in Synthesis Example 1 except that the added amount of the propylene oxide was 2.53 kg, the addition time of the second methyl chloride was 82 minutes, and the addition time of the propylene oxide was 17 minutes.

Regarding the obtained HPMC, a degree of substitution (DS) of methoxy groups and a molar substitution (MS) of hydroxypropoxy groups, the viscosity at 20°C of the 2% by mass aqueous solution, the molar fraction (MF at 3-position) and the ratio of (MF at 3-position) to MS are shown in Table 1.

In a separate experiment for sampling under the same conditions, the reaction percantageof the propylene oxide was 91.2% at the time when the total reaction percentage of the first and second methyl chlorides was 50%.

### Comparative Synthesis Example 2

HPMC was synthesized, pulverized and depolymerized in the same manner as in Synthesis Example 5 except that the added amount of the propylene oxide was 1.60 kg, the addition time of the second methyl chloride was 80 minutes, and the addition time of the propylene oxide was 10 minutes. The results are shown in Table 1.

In a separate experiment for sampling under the same conditions, the reaction percentage of the propylene oxide was 92.4% at the time when the total reaction percentage of the first and second methyl chloride was 50%.

**Table 1**

| | DS | MS | viscosity of 2% by mass aq. solution (mPa.s) | MF at 3-position | ratio of MF at 3-position to MS | substitution degree type |
|---|---|---|---|---|---|---|
| Syn.Ex.1 | 1.90 | 0.237 | 5.67 | 0.029 | 0.122 | 2910 |
| Syn.Ex.2 | 1.91 | 0.241 | 6.21 | 0.042 | 0.174 | 2910 |
| Syn.Ex.3 | 1.91 | 0.241 | 4.33 | 0.042 | 0.174 | 2910 |
| Syn.Ex.4 | 1.91 | 0.241 | 3.04 | 0.042 | 0.174 | 2910 |
| Syn.Ex.5 | 1.90 | 0.161 | 4.52 | 0.028 | 0.174 | 2906 |
| Comp.Syn.Ex.1 | 1.90 | 0.240 | 5.84 | 0.026 | 0.108 | 2910 |
| Comp.Syn.Ex.2 | 1.89 | 0.161 | 4.45 | 0.017 | 0.106 | 2906 |

### EXAMPLE 1

The HPMC produced in Synthesis Example 1 was used to produce liquid compositions by the following methods, and then transmittance values of the liquid compositions were evaluated. The results are as shown in Table 2.

### <Production of Liquid Composition Containing 8:2 (wt/wt) Ethanol/Purified Water>

The 73.6 g of ethanol was weighed in a wide-mouthed 250 mL reagent bottle, and was being stirred with a stirrer (ZZ-1000 produced by Tokyo Rikakikai Co., Ltd.) having a stirring blade, while adding 8.0 g of HPMC thereto. Then, the resulting mixture was subjected to addition of 18.4 g of purified water, and stirred at 400 rpm at room temperature for 2 hours to produce a liquid composition.

### <Production of Liquid Composition Containing 5.5:4.5 (vol/vol)

### Dichloromethane/Ethanol>

The 110 ml (137.5 g) of dichloromethane and 90 ml (71.4 g) of ethanol were weighed in a wide-mouthed 300 mL reagent bottle, and were being stirred with a stirrer (ZZ-1000 produced by Tokyo Rikakikai Co., Ltd.) having a stirring blade, while adding 10.0 g of HPMC thereto; and stirred at 400 rpm at room temperature for 2 hours to produce a liquid composition.

### <Measurement of Transmittance>

The transmittance of each liquid composition produced above was measured using a photoelectric colorimeter (PC-50 type: produced by Kotaki Seisakusho Co., Ltd.) under the conditions of 720 nm filter and cell length of 20 mm.

### EXAMPLE 2

Liquid compositions were produced in the same manner as in Example 1 except that the HPMC produced in Synthesis Example 2 was used, and then transmittance values of the liquid compositions were evaluated. The results are as shown in Table 2.

### EXAMPLE 3

Liquid compositions were produced in the same manner as in Example 1 except that the HPMC produced in Synthesis Example 3 was used, and then transmittance values of the liquid compositions were evaluated. The results are as shown in Table 2.

### EXAMPLE 4

Liquid compositions were produced in the same manner as in Example 1 except that the HPMC produced in Synthesis Example 4 was used, and then transmittance values of the liquid compositions were evaluated. The results are as shown in Table 2.

### EXAMPLE 5

Liquid compositions containing ethanol and purified water were produced in the same manner as in Example 1 except that 68.0 g of ethanol, 17.0 g of purified water and 15.0 g of the HPMC produced in the Synthesis Example 1 were used, and then transmittance values of the liquid compositions were evaluated. The results are as shown in Table 2.

### Comparative Example 1

Liquid compositions were produced in the same manner as in Example 1 except that the HPMC produced in Comparative Synthesis Example 1 was used, and then transmittance values of the liquid compositions were evaluated. The results are as shown in Table 2.

### Comparative Example 2

Liquid compositions containing ethanol and purified were produced in the same manner as in Example 5 except that the HPMC produced in Comparative Synthesis Example 1 was used, and then transmittance values of the liquid compositions were evaluated. The results are as shown in Table 2.

**Table 2**

| | HPMC | liquid composition | | | |
|---|---|---|---|---|---|
| | | 8:2 (wt/wt) ethanol/ purified water solution of HPMC | | 5.5:4.5 (vol/vol) dichloromethane! ethanol solution of HPMC | |
| | | concentration of HPMC | transmittance | concentration of HPMC | transmittance |
| | | (% by mass) | (%) | (% by mass) | (%) |
| Example 1 | Syn.Ex.1 | 8 | 91 | 4.6 | 93 |
| Example 2 | Syn.Ex.2 | 8 | 93 | 4.6 | 94 |
| Example 3 | Syn.Ex.3 | 8 | 94 | 4.6 | 95 |
| Example 4 | Syn.Ex.4 | 8 | 92 | 4.6 | 95 |
| Example 5 | Syn.Ex.1 | 15 | 70 | - | - |
| Comp.Ex.1 | Comp.Syn.Ex.1 | 8 | 70 | 4.6 | 83 |
| Comp.Ex.2 | Comp.Syn.Ex.1 | 15 | 32 | - | - |

### EXAMPLE 6

The HPMC produced in Synthesis Example 5 was used to produce liquid compositions by the following methods, and the transmittance values of the liquid compositions were evaluated in the same manner as in Example 1. The results are shown in Table 3.

### <Production of Liquid Composition Containing 8:2 (wt/wt) Ethanol/Purified Water>

A liquid composition was produced in the same manner as in Example 1 except that the HPMC produced in Synthesis Example 5 was used.

### <Production of Liquid Composition Containing 7:3 (w/wt) Ethanol/Purified Water>

The 64.4 g of ethanol was weighed in a wide-mouthed 250 ml reagent bottle, and was being stirred with a stirrer (ZZ-1000 produced by Tokyo Rikakikai Co., Ltd.) having a stirring blade, while adding 8.0 g of the HPMC produced in Synthesis Example 5 thereto. Then, the resulting mixture was subjected to addition of 27.6 g of purified water, and stirred at 400 rpm at room temperature for 2 hours to produce a liquid composition.

### Comparative Example 3

Liquid compositions were produced in the same manner as in Example 6 except that the HPMC produced in Comparative Synthesis Example 2 was used, and the transmittance values of the liquid compositions were evaluated. The results are shown in Table 3.

**Table 3**

| | HPMC | liquid composition | | | |
|---|---|---|---|---|---|
| | | 8:2 (wt/wt) ethanol/ purified water solution of HPMC | | 7:3 (wt/wt) ethanol/ purified water solution of HPMC | |
| | | concentration of HPMC | transmittance | concentration of HPMC | transmittance |
| | | (% by mass) | (%) | (% by mass) | (%) |
| Example 6 | Syn.Ex.5 | 8 | 64 | 8 | 94 |
| Comp.Ex.3 | Comp.Syn.Ex.2 | 8 | 37 | 8 | 81 |

### EXAMPLE 7

The 35 g of HPMC produced in Synthesis Example 1 in 372 g of ethanol was stirred with a propeller-type stirrer to form a dispersion. The dispersion was subjected to addition of 93 g of purified wate and stirred for 1 hour for dissolving the HPMC to form a coating solution having a HPMC concentration of 7% by mass.

Uncoated tablets were produced by mixing 2 parts by mass of riboflavin (produced by Tokyo Tanabe Pharmaceutical Co., Ltd.), 90 parts by mass of lactose (Dilactose S produced by Freund Cooperation), 8 parts by mass of low-substituted hydroxypropyl cellulose (hydroxypropyl group substitution degree of 11% by mass), and 0.5 parts by mass of magnesium stearate, and tableting the resulting mixture with a rotary tableting machine (Virgo produced by KIKUSUI SEISAKUSHO Ltd.) under the conditions (tablet diameter: 8 mm, tableting pressure: 1t, the tableting preload: 0.3t, rotational speed:20 rpm, and the mass per tablet:200 mg).

The above-obtained coating solution was applied onto the uncoated tablets in the conditions described below so that 3 parts by mass of a coating layer was formed on 100 parts by mass of each uncoated tablet. Neither clogging of the filter through which the coating solution passed nor clogging of the spray nozzle was observed.
Apparatus: perforated pan coater (inner diameter: 33 cm)
Charge-in quantity: 1 kg
Intake air temperature: 57 to 62°C
Exhaust gas temperature: 38 to 40°C
Intake air flow: 1 m³/minute
Pan rotation speed: 18 rpm
Spraying rate: 5 to 6 g/minute
Spray air pressure: 150 kPa

As a result of visually observing the obtained coated tablets, it was confirmed that a good coating film had been formed.

It is evident from the results of Examples 1 to 6 and Comparative Examples 1 to 3 that HPMC having the ratio (MF at 3-position/MS) of 0.12 or more provided excellent solubility in comparison with HPMC having the same substitution degrees.

On the other hand, it is evident from the results of Examples 1 to 6 that 2910 types of HPMC (in Examples 1 to 5) having higher DS and MS provided the highest transmittance in comparison with HPMC having lower DS and MS. This is considered to be because effects of DS and MS on the solubility of HPMC in an organic solvent is large. Among the types of HPMC, 2910 and 2906 types are more preferred, and 2910 types are most preferred.

The reason why HPMC having the ratio (MF at 3-position/MS) of 0.12 or more provided excellent solubility is considered to be that intramolecular hydrogen bonding could be inhibited by replacing the hydroxy groups at 3-position carbons with hydroxypropoxy groups, where the hydroxy groups at 3-position carbons are considered to contribute to solubility in an organic solvent.

## Claims

1. Hydroxypropyl methyl cellulose having
a molar substitution (MS) of hydroxypropoxy groups of from 0.1 to 0.5;
a ratio (referred to as "MF at 3-position/MS") of 0.12 or more, as calculated by dividing a molar substitution (referred to as "MF at 3-position") of anhydroglucose units in which only a hydroxy group at the 3-position carbon is substituted with a hydroxypropoxy group and none of hydroxy groups at the 2-position and 6-position carbons is substituted with a hydroxypropoxy group by a molar substitution (referred to as "MS") of hydroxypropoxy groups; and
a viscosity at 20°C of from 1.0 to 50 mPa·s, as determined in a 2% by mass aqueous solution.

2. The hydroxypropyl methyl cellulose according to claim 1, wherein a degree of substitution (DS) of methoxy groups is from 1.0 to 2.2.

3. The hydroxypropyl methyl cellulose according to claim 1, wherein the molar fraction (referred to as "MF at 3-position") is 0.027 or more.

4. A liquid composition comprising:
hydroxypropyl methyl cellulose according to any one of claims 1 to 3, and
an organic solvent.

5. The liquid composition according to claim 4, wherein a content of the hydroxypropyl methyl cellulose is more than 0% by mass and not more than 20% by mass.

6. The liquid composition according to claim 4, wherein the organic solvent is miscible with water and the liquid composition further comprises water.

7. The liquid composition according to claim 4, further comprising an active ingredient.

8. A method for producing a preparation, the method comprising a step of coating a core comprising an active ingredient with the liquid composition of claim 4.

9. A method for producing a film, the method comprising a step of applying the liquid composition of claim 4 on a substrate, and removing the solvent from the applied liquid composition to obtain a film.

10. The method for producing a film according to claim 9, further comprising a step of removing the film from the substrate.

11. A method for producing a solid dispersion, the method comprising a step of spray-drying the liquid composition of claim 7 to obtain a solid dispersion.

## Patentansprüche

1. Hydroxypropylmethylcellulose mit
einer molaren Substitution (MS) von Hydroxypropoxygruppen von 0,1 bis 0,5;
einem Verhältnis (als "MF an 3-Position/MS" bezeichnet) von 0,12 oder mehr bei Berechnung durch Teilen einer molaren Substitution (als "MF an 3-Position" bezeichnet) von Anhydroglucose-Einheiten, bei der nur eine Hydroxygruppe an dem Kohlenstoff in der 3-Position durch eine Hydroxypropoxygruppe substituiert ist und keine der Hydroxygruppen an den Kohlenstoffen in der 2-Position und der 6-Position durch eine Hydroxypropoxygruppe substituiert ist, durch eine molare Substitution (als "MS" bezeichnet) von Hydroxypropoxygruppen; und
einer Viskosität bei 20 °C von 1,0 bis 50 mPa·s bei Bestimmung in einer 2 massen-%igen wässrigen Lösung.

2. Hydroxypropylmethylcellulose nach Anspruch 1, wobei ein Grad der Substitution (DS) von Methoxygruppen 1,0 bis 2,2 beträgt.

3. Hydroxypropylmethylcellulose nach Anspruch 1, wobei die Molfraktion (als "MF an 3-Position" bezeichnet) 0,027 oder mehr beträgt.

4. Flüssige Zusammensetzung, umfassend:
Hydroxypropylmethylcellulose nach einem der Ansprüche 1 bis 3 und
ein organisches Lösungsmittel.

5. Flüssige Zusammensetzung nach Anspruch 4, wobei ein Gehalt der Hydroxypropylmethylcellulose mehr als 0 Massen-% und nicht mehr als 20 Massen-% beträgt.

6. Flüssige Zusammensetzung nach Anspruch 4, wobei das organische Lösungsmittel mit Wasser mischbar ist und die flüssige Zusammensetzung ferner Wasser umfasst.

7. Flüssige Zusammensetzung nach Anspruch 4, ferner umfassend einen Wirkstoff.

8. Verfahren zum Herstellen einer Zubereitung, wobei das Verfahren einen Schritt des Beschichtens eines Kerns, der einen Wirkstoff umfasst, mit der flüssigen Zusammensetzung nach Anspruch 4 umfasst.

9. Verfahren zum Herstellen einer Folie, wobei das Verfahren einen Schritt des Aufbringens der flüssigen Zusammensetzung nach Anspruch 4 auf ein Substrat und des Entfernens des Lösungsmittels von der aufgebrachten flüssigen Zusammensetzung unter Erhalt einer Folie umfasst.

10. Verfahren zum Herstellen einer Folie nach Anspruch 9, ferner umfassend einen Schritt des Entfernens der Folie von dem Substrat.

11. Verfahren zum Herstellen einer festen Dispersion, wobei das Verfahren einen Schritt des Sprühtrocknens der flüssigen Zusammensetzung nach Anspruch 7 unter Erhalt einer festen Dispersion umfasst.

## Revendications

1. Hydroxypropylméthylcellulose ayant
une substitution molaire (MS) de groupes hydroxypropoxy de 0,1 à 0,5 ;
un rapport (appelé « MF en position 3/MS ») de 0,12 ou plus, comme calculé en divisant une substitution molaire (appelée « MF en position 3 ») d'unités anhydroglucose dans lesquelles seulement un groupe hydroxy sur le carbone en position 3 est substitué par un groupe hydroxypropoxy et aucun des groupes hydroxy sur les carbones en position 2 et 6 n'est substitué par un groupe hydroxypropoxy par une substitution molaire (appelée « MS ») de groupes hydroxypropoxy ; et
une viscosité à 20 °C allant de 1,0 à 50 mPa·s, comme déterminée dans une solution aqueuse à 2 % en masse.

2. Hydroxypropylméthylcellulose selon la revendication 1, dans laquelle le degré de substitution (DS) de groupes méthoxy est de 1,0 à 2,2.

3. Hydroxypropylméthylcellulose selon la revendication 1, dans laquelle la fraction molaire (appelée « MF en position 3 ») est de 0,027 ou plus.

4. Composition liquide comprenant :
une hydroxypropylméthylcellulose selon l'une quelconque des revendications 1 à 3, et
un solvant organique.

5. Composition liquide selon la revendication 4, dans laquelle une teneur de l'hydroxypropylméthylcellulose est supérieure à 0 % en masse et non supérieure à 20 % en masse.

6. Composition liquide selon la revendication 4, dans laquelle le solvant organique est miscible à l'eau et la composition liquide comprend en outre de l'eau.

7. Composition liquide selon la revendication 4, comprenant en outre un ingrédient actif.

8. Procédé de production d'une préparation, le procédé comprenant une étape d'enrobage d'un noyau comprenant un ingrédient actif avec la composition liquide selon la revendication 4.

9. Procédé de production d'un film, le procédé comprenant une étape d'application de la composition liquide selon la revendication 4 sur un substrat, et d'élimination du solvant de la composition liquide appliquée pour obtenir un film.

10. Procédé de production d'un film selon la revendication 9, comprenant en outre une étape d'élimination du film du substrat.

11. Procédé de production d'une dispersion solide, le procédé comprenant une étape de séchage par pulvérisation de la composition liquide selon la revendication 7 pour obtenir une dispersion solide.
